(19) Europäisches Patentamt
European Patent Office
Office européen des brevets

(11) Publication number: 0 177 673 B1

# (12) EUROPEAN PATENT SPECIFICATION

(45) Date of publication of patent specification: 28.08.91

(51) Int. Cl.⁵: C07C 35/18, C07C 29/48, A61K 31/045

(21) Application number: 85107580.4

(22) Date of filing: 19.06.85

## (54) Mucosecretolytic pharmaceutical compositions containing alpha, alpha-dimethyl-5-hydroxy-3-cyclohexene-1,4 dimethanol.

(30) Priority: 08.08.84 IT 2225884

(43) Date of publication of application:
16.04.86 Bulletin 86/16

(45) Publication of the grant of the patent:
28.08.91 Bulletin 91/35

(84) Designated Contracting States:
AT BE CH DE FR GB IT LI LU NL SE

(56) References cited:
CH-A- 549 006
FR-A- 2 086 134

XENOBIOTICA, vol. 13, no. 3, 1983, pages 139-146; P. VENTURA et al.: "The metabolism of trans-sobrerol in the rat"

BULLETIN DE LA SOCIETE CHIMIQUE DE FRANCE, no. 12, 1971, page 4391-4396, no. 744; Y. BESSIERE-CHRETIEN et al.: "Action de l'aluminohydrure de lithium et du diborane sur deux epoxyalcools"

(73) Proprietor: CAMILLO CORVI S.p.A.
via S. Marco 18
Milan(IT)

(72) Inventor: Corvi Mora, Camillo
via dei Mille 3
Piacenza(IT)

(74) Representative: Klausner, Erich et al
c/o Ufficio Internazionale Brevetti Ing. C. Gregorj S.p.A. Via Dogana 1
I-20123 Milano(IT)

EP 0 177 673 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention concerns pharmaceutical compositions having mucosecretolytic activity, which contain α,α-dimethyl-5-hydroxy-3-cyclohexene-1,4-dimethanol having the structural formula

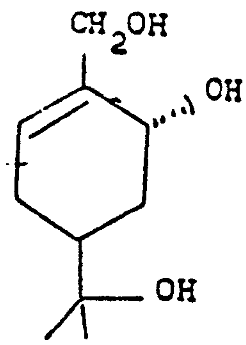

(I)

Code CO/1470 $C_{10}H_{18}O_3$ mol.wt. 186.24; m.p.114-116°C.

Unexpectedly, the compound of formula (I) has shown a very interesting pharmacological activity as a mucolytic agent for bronchial secretion, which causes the product of formula (I) to constitute potentially a novel drug intended for the therapy of acute as well as chronic diseases of the bronchopneumonic apparatus.

The compound of formula (I) is known from XENOBIOTICA, Vol.13,No.3(1983), wherein its formula is illustrated in Fig.8 at p.145; the compound is identified therein as metabolite M5. The cited reference teaches that trans-sobrerol,viz. dl-trans-α,α-4-trimethyl-3-cyclohexene-5-hydroxy -1-methanol, - a known mucofluidifying agent -is extensively metabolized in vivo into seven different metabolites,one of which is α,α-dimethyl-5-hydroxy-3-cyclohexene-1,4-dimethanol. The prior art,however, does not teach that metabolite M5, or any of the other metabolites,has any therapeutic activity.

The compound of formula (I) can be prepared according to the following reaction scheme:

SYNTHESIS SCHEME OF CO/1470

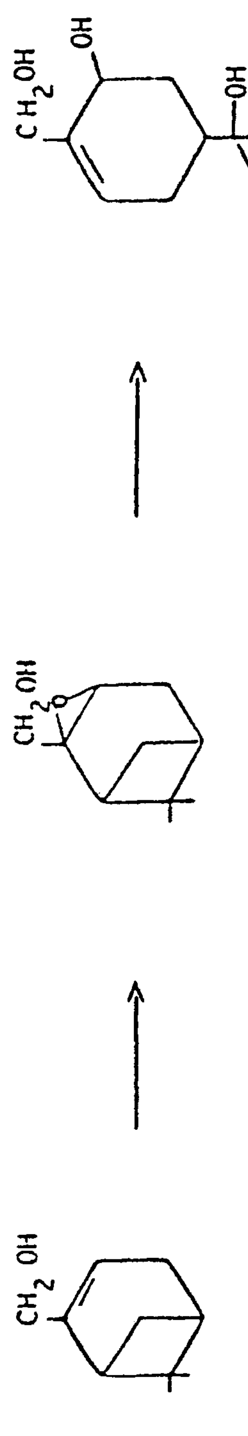

(III) $C_{10}H_{16}O$ mol. wt. 152.24

(II) $C_{10}H_{16}O_2$ mol.wt.168.235

(I) $C_{10}H_{18}O_3$ mol.wt.~186.24

The compound of formula (III) : (-)-6,6-dimethylbicyclo [3.3.1.]-ept-2-ene-methanol (also known as (-)myrtenol: see 1981-1982 Aldrich-Europe Catalog Handbook of Fine Chemicals, No.18,841-7) can form an epoxide by treatment with per-acids such as m-chlorobenzoic acid or peracetic acid (40% acetic solution). The epoxide is obtained when, in particular, peracetic acid is employed, in a medium of anhydrous methylene chloride (ethanol free), in the presence of anhydrous sodium carbonate which acts as an acid acceptor. In the reaction medium there forms, concurrently with the epoxidation,an equimolar salt mixture of anhydrous sodium bicarbonate and sodium acetate which can be eliminated by filtration or by water dissolution and separation of the chloromethylene solution of the formed epoxide. The organic

chloromethylene solution as separated is then concentrated by evaporation under reduced pressure, to give the concentrated epoxide.

The obtainment of the epoxide of (-)-6,6-dimethylbicyclo [3.3.1.]ept-2-ene-2-methanol of formula (II) has led to make investigations on the hydration products, which may be obtained in an aqueous medium, in the presence of either diluted strong acids (1% solution of $H_2SO_4$, $H_3PO_4$) or weak acids (such as $H_2CO_3$, $H_2SO_3$) at temperatures from 1 to 20° C. The compound of formula (I) is thus obtained.

The intermediate compound of formula (II) - which is also known from Bulletin de la Société Chimique de France, No.12 (1971) - is subjected to hydration at a temperature of from 1 to 20° C in a weakly acidic aqueous medium, the reaction mixture is thus extracted with ethyl acetate in the presence of a sodium chloride solution, and then, upon concentration of the organic phase, the compound of formula (I) is obtained, in crystalline form.

The above described reaction steps are readily derived from FR-A-2 086 134 and CH-A-549 006. The first of these two patents concerns a process for preparing sobrerol and the pharmaceutical use thereof. According to said process, sobrerol is prepared by a two-step method. In the first step (epoxidation), α-pinene is oxydized using a 15-30% perbenzoic acid chloroform solution at a temperature of from -1° C to -6° C, in 4 to 8 hours. In the second step, the pinene epoxide is hydrated, at 36 - 100° C, in an aqueous solution of carbon anhydride.

The second of the above referred patents concerns a process for preparing α-pinene oxide.

Example

To a solution of 30 g (-)-6,6-dimethylbicyclo[.3.3.1.]-ept-2-ene-2-methanol in 300 ml methylene chloride, 34 g of anhydrous sodium carbonate is added. The resulting mixture is cooled to a temperature of from 5 to 10° C and 60 ml of 40% peracetic acid, under Vigorous stirring, is added thereto. At the end of the addition, the cooling is interrupted and the mixture is left under stirring at room temperature over 12 hours.

The reaction mixture is diluted with water, the organic phase is separated, washed with water, desiccated and evaporated to dryness; 31.5 g (95%) of epoxide is so obtained.

To the above compound 60 m1 of water and 10 g of solid carbon dioxide are added and the resulting mixture is stirred vigorously over 3 hours, diluted with water saturated with sodium chloride and extracted repeatedly with ethyl acetate. The combined organic phases are washed with a little water, anhydrated and concentrated to a volume of about 100 ml. The precipitate is suction filtered and 14 g (38%) of a white crystalline product,m.p.114-116° C, is thus obtained.

Referring to the activity as a mucosecretolytic agent shown by the compound of formula (I), the present invention thus provides pharmaceutical compositions which contain the compound of formula (I) in dosage unit.

The compound of formula (I) - CO/1470 - prepared as described above,corresponds to the following analytical data:

Elemental analysis

| | | | |
|---|---|---|---|
| Calculated | C = 64.49 % | H = 9.74 % | O = 25.77 % |
| Found | C = 64.37 % | H = 9.92 % | |
| | C = 64.40 % | H = 9.94 % | |
| | C = 64.35 % | H = 9.56 % | |

I.R.(nujol dispersion; $cm^{-1}$):
3290 ϑ OH
1140; 1057; 1025; 1011; 1005; 963; 930; 835; characteristic bands

N.M.R. ($D_2O$ solvent; D.S.S. reference; δppm):

| | | |
|---|---|---|
| 6.0 | centre | c.a.(1 H; = CH) |
| 4.33 | centre | c.a.(1 H; <u>CH</u>-OH; $W_{\frac{1}{2}}$ = 7,5 $H_2$) |
| 4.12 | | c.a.(2 H; $\underline{CH_2}$-OH) |
| 2.43 ÷ 1.32 | | c.a.(5 H; $\underline{CH_2}$-<u>CH</u>-$\underline{CH_2}$) |
| 1.22 | | s. (6 H; gem. $CH_3$) |

c.a. = complex absorption $W_{\frac{1}{2}}$ = broadness at half height

s. = singlet

D.S.S. = 3(trimethylsilyl) propane-sulfonic acid, sodium salt

M.S.(Quadrupole; electronic impact,direct insertion,80eV, 70 mA, m/z):
168[$(M-18)^+$, 1%]; 153 [$(m-18-15)^+$ 3%]; 151 (2%); 150 (10%); 137 (8%); 135 (13%); 126 (11%); 122 (4%); 114 (11%); 110 (10%); 109 (9%); 108 (17%); 107 (16%); 106 (5%); 105 (3%); 97 (5%); 96 (9%); 95 (41%); 94 (5%); 93 (12%); 92 (23%); 91 (22%); 82 (9%); 81 (27%); 80 (9%); 79 (44%); 78 (11%); 77 (15%); 69 (17%); 67 (16%); (base peak)

Table 1 - <u>Bronchosecretagogue activity of CO/1470</u>

Below are reported the average values of percent increase of the bronchial mucus secretion upon treatment with CO/1470 and other known standards as compared to the basal values. Further, there is reported the number of rabbits which showed an increase of bronchial secretion as compared with all the animals treated with CO/1470 at the various doses and via the two administration routes as utilized.

| Dose in mg/Kg | Administration route | Bronchial secretion average increase, % | N° of rabbits with a secretion increase/N° of rabbits CO-treated |
|---|---|---|---|
| CO/1470 | | | |
| 25 | oral | 42 | 3/10 |
| 100 | oral | 54 | 8/11 |
| 400 | oral | 171 | 10/10 |
| 2,5 | intravenous | 20 | 4/10 |
| 5 | intravenous | 59 | 7/10 |
| 10 | intravenous | 72 | 9/10 |
| N-acetylcysteine | | | |
| 400 | oral | 22 | 8/16 |
| 600 | oral | 59.4 | 6/9 |
| Bromhexine | | | |
| 200 | oral | 35.8 | 4/8 |
| 400 | oral | 43 | 6/8 |
| Carboxymethylcysteine | | | |
| 200 | oral | 10 | 4/10 |
| 400 | oral | 46 | 5/10 |

The pharmaceutical forms containing the above mentioned active ingredients are those suitable for intramuscular and intravenous administration, those suitable for aerosol as well as the ones for oral administration, in particular: capsules, tablets, granular forms in sachets, syrups; contemplated are also those for rectal administration such as suppositories.

In the forms as mentioned, conventional excipients are combined with the compound of formula (I).

In the solid oral forms (tablets, capsules, granular forms), the preferred excipients are: lactose, starch, cellulose and its derivatives, with all the carrier materials for the preparation of the pharmaceutical form such as precipitated silica, talc, calcium or magnesium stearate.

In the form of syrup, the active compound is dissolved in a sugar solution (saccharose, glucose, sorbitol) with addition of aromatizing and preserving agents.

In the form of suppositories, the main excipient consists of triglycerides of fatty acids, either pure or as a mixture with oxyethylated derivatives.

In the injectable or aerosol forms, the compound of formula (I) is brought to an isotonic solution and either cold or hot sterlized.

**Claims**

**Claim for the following Contracting States: BE, CH, DE, FR, GB, IT, LI, LU, NL, SE**

1. Pharmaceutical compositions having mucosecretolytic activity, which contain the compound of formula (I)

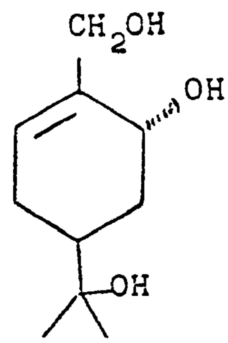

(I)

with the addition of the pharmaceutically acceptable excipients, carriers, and auxiliary materials, for the various pharmaceutical forms.

**Claim for the following Contracting State: AT**

1. A process for making pharmaceutical compositions having mucosecretolytic activity, characterised in that the compound of formula (I) :

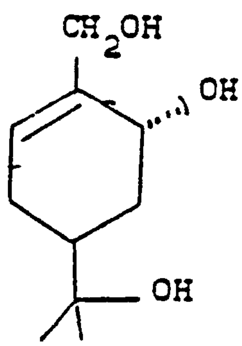

(I)

is admixed with one ore more pharmaceutically acceptable excipients, carriers, and auxiliary material, suitable for the various pharmaceutical forms.

**Revendications**

**Revendication pour les Etats contractants suivants: BE, CH, DE, FR, GB, IT, LI, LU, NL, SE**

1. Compositions pharmaceutiques ayant une activité mucosécrétolique, contenant le composé de formule (I)

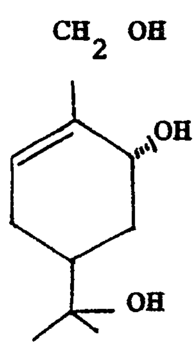

(I)

additionné d'excipients, véhicules et aides de formulation convenable, acceptable en pharmacie, pour les différentes formes pharmaceutiques.

**Revendication pour l'Etat contractant suivant: AT**

1. Procédé pour la préparation d'une composition pharmaceutique caractérisé en ce qu'on mélange le composé de formule (I) :

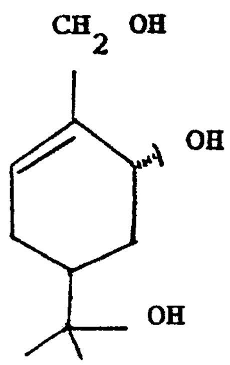

(I)

ayant une activité mucosécrétolique, avec les excipients, les véhicules et les aides de formulation convenable, acceptable en pharmacie, à donner une forme d'administration convenable.

**Patentansprüche**
**Patentanspruch für folgende Vertragsstaaten: BE, CH, DE, FR, GB, IT, LI, LU, NL, SE**

1. Pharmazeutische Zubereitungen mit mucosekretolytischer Wirkung, die die Verbindung der Formel (I)

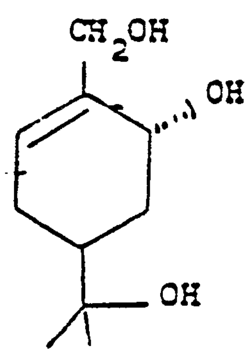

(I)

und die geeigneten pharmazeutisch unbedenklichen Zusatzmittel, Träger und Hilfsmittel für die verschiedenen Verabreichunsformen enthält.

**Patentanspruch für folgenden Vertragsstaat: AT**

1. Verfahren zur Herstellung von pharmazeutischen Zubereitungen mit mucosekretolytischer Wirkung dadurch gekennzeichnet, dass die Verbindung der Formel (I)

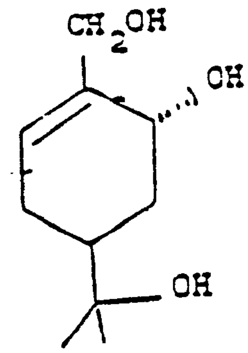

(I)

mit den geeigneten, pharmazeutische unbedenklichen Zusatzmitteln, Trägern und Hilfsmitteln für die verschiedenen Verabreichungsformen verarbeitet wird.